# EUROPÄISCHE PATENTSCHRIFT

(11) **EP 1 401 811 B1**
(45) Veröffentlichungstag und Bekanntmachung des Hinweises auf die Patenterteilung: **15.06.2005**
(21) Anmeldenummer: 02754703.3
(22) Anmeldetag: 18.06.2002
(51) Int. Cl.: C07D 207/267, C07D 201/08

(54) **VERFAHREN ZUR HERSTELLUNG VON PYRROLIDONEN**
METHOD FOR PRODUCING PYRROLIDONES
PROCEDE POUR LA PRODUCTION DE PYRROLIDONES

(30) Priorität: 19.06.2001 DE 10129336
(43) Veröffentlichungstag der Anmeldung: 31.03.2004
(73) Patentinhaber: BASF AKTIENGESELLSCHAFT, 67056 Ludwigshafen (DE)
(72) Erfinder: FISCHER, Rolf-Hartmuth, 69121 Heidelberg (DE); PINKOS, Rolf, 67089 Bad Dürkheim (DE); RÖSCH, Markus, 55276 Oppenheim (DE); STEIN, Frank, 67098 Bad Dürkheim (DE)
(74) Vertreter: Isenbruck, Günter, Dr.
(86) Internationale Anmeldenummer: PCT/EP2002/006722
(87) Internationale Veröffentlichungsnummer: WO 2002/102773

(56) Entgegenhaltungen:
- EP-A- 0 349 119
- EP-A- 0 545 150
- WO-A-93/16042
- WO-A-97/24346
- DE-A- 19 626 123
- US-A- 4 851 546
- DATABASE CASREACT [Online] Chemical Abstracts Services, Columbo, Ohio, US; OTAKE, MASAYUKI ET AL: "Preparation of lactams" retrieved from STN Database accession no. 109:170226 XP002212029 & JP 63 027476 A (MITSUBISHI CHEMICAL INDUSTRIES CO) 5. Februar 1988 (1988-02-05) in der Anmeldung erwähnt

## Beschreibung

Die vorliegende Erfindung betrifft ein Verfahren zur Herstellung von Pyrrolidonen. Die Synthese geschieht durch katalytische Hydrierung in der Gasphase von Substraten, die ausgewählt sind aus der Gruppe bestehend aus Derivaten der Maleinsäure und Bernsteinsäure sowie diesen Säuren selbst. In diesen Substraten kann der in dem Pyrrolidon vorliegende Stickstoffbaustein bereits vorhanden sein, gegebenenfalls werden Ammoniak oder primäres Amin bei der Synthese zugegeben, sollte dieser Stickstoffbaustein nicht vorliegen. Das erfindungsgemäße Verfahren gestattet es dabei, Pyrrolidone herzustellen, die gewünschtenfalls N-alkyliert sind, die Pyrrolidone können jedoch unabhängig davon auch einen oder mehrere Alkylsubstituenten an den Kohlenstoffatomen des Rings aufweisen.

Die Herstellung von Pyrrolidonen durch Hydrierung von Maleinsäureanhydrid (MSA) oder auch Bernsteinsäureanhydrid (BSA) bzw. den entsprechenden offenkettigen Säuren oder Estern in Gegenwart von Ammoniak oder primären Aminen ist an sich bekannt.

So beschreibt die EP-A 745 589 ein Verfahren zur Herstellung von gegebenenfalls N-substituierten Pyrrolidonen, bei dem MSA, Ammoniak oder ein primäres Amin und Wasserstoff an einem Trägerkatalysator miteinander umgesetzt werden. Der Katalysator enthält sowohl Rhenium als auch Palladium in metallischer oder gebundener Form.

Es konnte gezeigt werden, daß bei der Hydrierung von MSA zu Tetrahydrofuran zunächst BSA und anschließend γ-Butyrolacton (GBL) entstehen. Diese Produkte können durch weitere Hydrierung zu 1,4-Butandiol (BDO) und schließlich zu Tetrahydrofuran (THF) umgesetzt werden.

Werden die vorstehend genannten Edukte daher unter hydrierenden Bedingungen mit Ammoniak oder primären Aminen umgesetzt, laufen eine Vielzahl konkurrierender Reaktionen ab, wodurch die Selektivität zu den gewünschten Pyrrolidonen im allgemeinen sehr niedrig ist.

Zudem enthalten die dabei eingesetzten Katalysatoren häufig Chrom, generell in Form von Chromoxid. Aufgrund der Toxizität von Chrom ist jedoch die Entwicklung von Katalysatoren erwünscht, die ohne Anwesenheit von Chrom gute Ausbeuten und Selektivitäten im Hinblick auf das gewünschte Pyrrolidon aufweisen.

Ein weiterer Nachteil der bis jetzt eingesetzten Verfahren der Herstellung von Pyrrolidonen bzw. der darin angesetzten Katalysatoren besteht darin, daß als Edukt generell vorgereinigtes MSA bzw. ein Derivat davon benutzt werden muß. Das Edukt muß daher nach seiner Herstellung in häufig aufwendigen Verfahren von Verunreinigungen befreit werden. MSA wird hergestellt durch partielle Oxidation von bestimmten Kohlenwasserstoffen, nämlich Benzol, Butengemischen oder n-Butan, wobei dieses letztere vorzugsweise eingesetzt wird. Das Rohprodukt der Oxidation enthält neben dem gewünschten MSA vor allem Nebenprodukte wie Wasser, Kohlenmonoxid, Kohlendioxid, nicht umgesetzten Ausgangskohlenwasserstoff sowie Essig- und Acrylsäure, wobei diese Nebenprodukte bei Verwendung aller vorstehend genannten Kohlenwasserstoffe entstehen. Zumeist werden die Nebenprodukte durch aufwendige Verfahren abgetrennt, beispielsweise durch Destillation. Dieses Reinigen erweist sich insbesondere als notwendig, weil die bei der Herstellung von Pyrrolidon aus MSA unter Zusatz von Ammoniak oder Amin unter hydrierenden Bedingungen eingesetzten Katalysatoren generell empfindlich auf solche Verunreinigungen reagieren. Die Desaktivierung der Katalysatoren ist bereits bei Einsatz von gereinigtem MSA ein Problem, da durch Belegung mit Polymerisationsprodukten des MSA der Katalysator in der Regel in relativ kurzen Intervallen, die oftmals bei ca. 100 Stunden liegen, regeneriert werden muß. Die Tendenz zur Desaktivierung ist beim Vorliegen polymerisierbarer Verbindungen, beispielsweise Acrylsäure, noch erhöht.

Die EP-A 545 150 offenbart ein Verfahren zur Herstellung von N-organylsubstituierten Pyrrolidonen, insbesondere N-Methylpyrrolidon, ausgehend von entsprechenden Dicarbonsäurederivaten. Dabei wird ein Katalysator eingesetzt, der mindestens ein

Element der ersten, siebten oder achten Nebengruppe des Periodensystems der Elemente enthält. Als Stickstoffbaustein kann ein primäres Amin mit dem gewünschten Organylsubstituenten eingesetzt werden. Es ist aber auch möglich, ein Gemisch eines entsprechenden sekundären und/oder tertiären Amins mit dem primären Amin einzusetzen. Die Reaktion wird unter Zusatz von Wasser und/oder Ammoniak durchgeführt. In einem Beispiel wird MSA mit Methylamin und Wasserstoff unter Zusatz von Wasser zu N-Methylpyrrolidon bei 200 bar umgesetzt, wobei ein Katalysator enthaltend 50 Gew.-% CuO und 50 Gew.-% Al₂O₃ eingesetzt wird. Die Ausbeute beträgt nur 38 %.

Die JP 63-27476 offenbart ein Verfahren zur Herstellung von Pyrrolidonen durch Gasphasenhydrierung von Imiden der Maleinsäure oder Bernsteinsäure, vorzugsweise der Bernsteinsäure. Es wurden Katalysatoren auf Cu-Basis eingesetzt, die ein Oxid von Cr, Mg oder Zn enthalten können. Es werden mit dem Verfahren Pyrrolidon-Ausbeuten von maximal 66 % (nach den Beispielen) erzielt, wobei jedoch immer ein Gemisch aus Succinimid in Butyrolacton im Verhältnis 20:80 eingesetzt wird.

WO-A-9724346 beschreibt ein Verfahren zur Herstellung von Butyrolacton aus Maleinsäureanhydrid durch Hydrierung in der Gasphase unter Verwendung eines CuO/Al2O3-Katalysators. Das so erhaltene Butyrolacton kann zu Pyrrolidonen weiterverarbeitet werden. Allerdings wird hierzu ein Hochdruckverfahren in flüssiger Phase vorgeschlagen.

EP-A-349119 beschreibt die Reaktion von Butyrolacton in der Gasphase zu Pyrrolidon (siehe Seite 3 Zeile 39-42, Tabelle II Beispiel 7).

WO-A-9316042 beschreibt eine Gasphasenhydrierung von 1,4-Dicarbonsäurederivaten zur Herstellung von Pyrrolidonen unter Verwendung von Cu/Cr-Katalysatoren.

Die Aufgabe der vorliegenden Erfindung besteht darin, ein Verfahren zur Verfügung zu stellen, das es gestattet, gegebenenfalls substituierte Pyrrolidone durch Umsetzung von Maleinsäureanhydrid oder einer verwandten Verbindung mit hohen Ausbeuten und Selektivitäten herzustellen. Die verwendeten Katalysatoren sollen dabei frei von Chrom sein und es ermöglichen, ein MSA bzw. eine damit verwandte Verbindung als Edukt zu verwenden, das bzw. die nicht aufwendig vorgereinigt werden muß. Vorzugsweise sollte ein MSA oder eine damit verwandte Verbindung in einer Qualität eingesetzt werden können, die direkt nach der Herstellung vorliegt.

Diese Aufgabe wird gelöst durch ein Verfahren zur Herstellung von gegebenenfalls N-substituiertem Pyrrolidon durch Hydrierung in der Gasphase unter wasserfreien Bedingungen eines Substrats, das ausgewählt ist aus C₄-Dicarbonsäuren und deren Derivaten, gegebenenfalls unter Zusatz von Ammoniak oder primären Aminen, unter Verwendung eines Cr-freien Katalysators, der 5 bis 95 Gew.-% CuO, vorzugsweise 30 bis 70 Gew.-% CuO, und 5 bis 95 Gew.-% Al₂O₃, vorzugsweise 30 bis 70 Gew.-% Al₂O₃, und 0 bis 60 Gew.-%, vorzugsweise 5 bis 40 Gew.-% ZnO aufweist.

Unter dem Begriff "C₄-Dicarbonsäuren und deren Derivate" werden im Bezug auf die vorliegende Anmeldung Maleinsäure und Bernsteinsäure, die gegebenenfalls einen oder mehrere C₁-C₆-Alkylsubstituenten aufweisen, sowie die Mono- und Diester, Anhydride und Imide dieser gegebenenfalls alkylsubstituierten Säuren verstanden. Beispiele umfassen Maleinsäuremonomethylester, Maleinsäuredimethylester, Maleinsäureanhydrid, Bernsteinsäureanhydrid, Citraconsäureanhydrid, Succinimid, N-Methylsuccinimid, N-Butylsuccinimid, Maleinimid und N-Methylmaleinimid. Für den Fall, daß Imide als Edukte eingesetzt werden, wird kein Ammoniak oder primäres Amin zugesetzt. Die Imide können dabei in einer vorgeschalteten Stufe durch Umsetzung von Ammoniak oder dem gewünschten primären Amin mit der C₄-Dicarbonsäure oder deren Derivat ohne Zusatz von Wasserstoff hergestellt worden sein.

Vorzugsweise werden in die erfindungsgemäße Reaktion die Anhydride, insbesondere Maleinsäureanhydrid oder Bernsteinsäureanhydrid, eingesetzt. Das meist bevorzugte Substrat ist Maleinsäureanhydrid (MSA).

In einer bevorzugten Ausführungsform wird das erfindungsgemäße Verfahren so ausgeführt, daß zunächst die C₄-Dicarbonsäure und/oder deren Derivat, vorzugsweise Maleinsäureanhydrid, mit Wasserstoff in Gegenwart der erfindungsgemäßen Katalysatoren hydriert wird. Ammoniak oder die jeweiligen Amine werden erst dann zugeführt, wenn die Kohlenstoff-Kohlenstoff-Doppelbindung der genannten Edukte überwiegend oder vollständig hydriert wurde und dementsprechend das Hydriergemisch überwiegend oder vollständig aus Bernsteinsäure, Bernsteinsäureestern und/oder Bernsteinsäureanhydrid besteht. Vorzugsweise wird dieses Verfahren in einem einzigen Reaktor durchgeführt, wobei Ammoniak oder das primäre Amin erst an einer Stelle bzw. zu einem Zeitpunkt in den Reaktor gegeben wird, an der bzw. dem die beschriebene Hydrierung des Edukts bereits eingetreten ist. Das erfindungsgemäße Verfahren kann dann aber auch in zwei Reaktoren durchgeführt werden, wobei die Hydrierung des Edukts in dem ersten Reaktor und die Umsetzung mit Ammoniak bzw. dem primären Amin in dem zweiten Reaktor erfolgt.

Der Stickstoffbaustein, der umgesetzt wird, ist im einfachsten Fall Ammoniak. Soll ein N-substituiertes Pyrrolidon hergestellt werden, so wird als Stickstoffbaustein ein primäres

Amin eingesetzt, das einen Substituenten aus der Gruppe bestehend aus gegebenenfalls substituierten cyclischen und acyclischen, verzweigten und unverzweigten aliphatischen Kohlenwasserstoff-Resten mit einer C-Zahl von 1 bis 20, vorzugsweise 1 bis 12 aufweist. Die Substituenten, die an dem aliphatischen Kohlenwasserstoff vorhanden sein können, sind vorzugsweise ausgewählt aus der Gruppe bestehend aus aromatischen Resten, dabei insbesondere dem Phenylrest, sowie Hydroxylgruppen, Halogenen und Alkoxyresten. Beispiele für bevorzugte primäre Amine sind Methylamin, Ethylamin, n-Propylamin, n-Butylamin, n-Decylamin, n-Dodecylamin, Cyclohexylamin, Benzylamin und Ethanolamin. Für den Fall, daß ein Imid als Edukt eingesetzt wird, kann dieses N-substituiert sein, wobei die Substituenten die vorstehend genannten Bedeutungen aufweisen können.

Anstelle von reinen primären Aminen können auch Gemische aus primären Amin mit den entsprechenden sekundären und tertiären Aminen, wie z.B. Methylamin, Dimethylamin und Trimethylamin verwendet werden. Diese Ausführungsform ist nicht bevorzugt. Durch dem Fachmann bekannte Maßnahmen wird dabei eine ausreichende Konzentration des primären Amins aufrechterhalten.

Die erfindungsgemäßen Hydrierkatalysatoren enthalten vor der Aktivierung mit Wasserstoff Kupferoxid und Aluminiumoxid oder Kupferoxid, Aluminiumoxid und Zinkoxid. Der Gehalt an Kupferoxid und Aluminiumoxid beträgt dabei 5 bis 95 Gew.-% Kupferoxid und 95 bis 5 Gew.-% Aluminiumoxid, bevorzugt 30 bis 70 Gew.-% Kupferoxid und 70 bis 30 Gew.-% Aluminiumoxid, besonders bevorzugt 50 bis 60 Gew.-% Kupferoxid und 50 bis 40 Gew.-% Aluminiumoxid. Derartige Katalysatoren können neben Kupferoxid und Aluminiumoxid in den angegebenen Mengen noch bis 60 Gew.-% Zinkoxid enthalten, vorzugsweise 5 bis 40 Gew.-%.

Optionsweise können die erfindungsgemäß verwendeten Katalysatoren, die Cr-frei sind, ein oder mehrere weitere Metalle oder eine Verbindung davon, vorzugsweise ein Oxid, aus den Gruppen 1 bis 14 (IA bis VIIIA und IB bis IVB der alten IUPAC-Nomenclatur) des Periodensystems der Elemente enthalten. Wird ein solches weiteres Oxid verwendet, wird vorzugsweise TiO₂, ZrO₂, SiO₂ und/oder MgO eingesetzt.

Es hat sich erwiesen, daß die erfindungsgemäß eingesetzten Katalysatoren hohe Selektivitäten zu den gewünschten Pyrrolidonen und hohe Ausbeuten liefern. Entgegen dem in der EP-A 545 150 beschriebenen Verfahren wird unter wasserfreien Bedingungen bearbeitet. Darunter versteht man, dass maximal nur das durch Hydrierung einer Carbonylgruppe und gegebenenfalls partielle Überhydrierung der zweiten Carbonylgruppe entstehende Wasser vorhanden ist und Wasser nicht gesondert zugesetzt wird. Lediglich die in den Edukten vorhandenen Verunreinigungen an Wasser können vorhanden sein.

Die eingesetzten Katalysatoren können zudem ein Hilfsmittel in einer Menge von 0 bis 10 Gew.-% enthalten. Unter Hilfsmittel versteht man organische und anorganische Stoffe, die zu einer verbesserten Verarbeitung während der Katalysatorherstellung und/oder zu einer Erhöhung der mechanischen Festigkeit der Katalysatorformkörper beitragen. Derartige Hilfsmittel sind dem Fachmann bekannt; Beispiele umfassen Graphit, Stearinsäure, Kieselgel und Kupferpulver.

Die Katalysatoren lassen sich nach dem Fachmann bekannten Methoden herstellen. Bevorzugt sind Verfahren, bei denen das Kupferoxid fein verteilt und innig vermischt mit den anderen Bestandteilen anfällt, besonders bevorzugt sind Fällungsreaktionen. Dabei werden in einem Lösungsmittel gelöste Vorläuferverbindungen in Gegenwart weiterer löslicher oder im Lösungsmittel suspendierter Metallverbindungen mit einem Fällungsmittel ausgefällt, abfiltriert, gewaschen, getrocknet und gegebenenfalls calciniert.

Diese Ausgangsmaterialien können nach bekannten Methoden zu den Formkörpern verarbeitet werden, beispielsweise Extrudieren, Tablettieren oder durch Agglomerationsverfahren, gegebenenfalls unter Zusatz von Hilfsmitteln.

Alternativ können erfindungsgemäße Katalysatoren beispielsweise auch durch Aufbringen der Aktivkomponente auf einen Träger hergestellt werden, beispielsweise durch Tränken oder Aufdampfen. Weiterhin können erfindungsgemäße Katalysatoren durch Verformen einer heterogenen Mischung aus Aktivkomponente oder Vorläuferverbindung hiervon mit einer Trägerkomponente oder Vorläuferverbindung hiervon erhalten werden.

Bei der erfindungsgemäßen Hydrierung, bei der neben MSA andere, vorstehend definierte C₄-Dicarbonsäuren oder deren Derivate als Edukt eingesetzt werden können, wird der Katalysator in reduzierter, aktivierter Form verwendet. Die Aktivierung erfolgt mit reduzierenden Gasen, vorzugsweise Wasserstoff oder Wasserstoff/Inertgas-Gemischen, entweder vor oder nach dem Einbau in den Reaktor, in dem das erfindungsgemäße Verfahren durchgeführt wird. Wurde der Katalysator in oxidischer Form in den Reaktor eingebaut, so kann die Aktivierung sowohl vor dem Anfahren der Anlage mit der erfindungsgemäßen Hydrierung als auch während des Anfahrens, also in situ, durchgeführt werden. Die separate Aktivierung vor dem Anfahren der Anlage erfolgt im allgemeinen mit reduzierenden Gasen, vorzugsweise Wasserstoff oder Wasserstoff/Inertgas-Gemischen bei erhöhten Temperaturen, vorzugsweise zwischen 100 und 300 °C. Bei der sogenannten in-situ-Aktivierung erfolgt die Aktivierung beim Hochfahren der Anlage durch Kontakt mit Wasserstoff bei erhöhter Temperatur.

Die Katalysatoren werden als Formkörper verwendet. Beispiele umfassen Stränge, Rippstränge, andere Extrudatformen, Tabletten, Ringe, Kugeln und Splitt.

Die BET-Oberfläche der Kupferkatalysatoren beträgt im oxidischen Zustand 10 bis 400 m²/g, vorzugsweise 15 bis 200 m²/g, insbesondere 20 bis 150 m²/g. Die Kupferoberfläche (N₂O-Zersetzung) des reduzierten Katalysators beträgt im Einbauzustand > 0,2 m²/g, vorzugsweise > 1 m²/g, insbesondere > 2 m²/g.

Gemäß einer Variante der Erfindung werden Katalysatoren verwendet, die eine definierte Porosität aufweisen. Diese Katalysatoren zeigen als Formkörper ein Porenvolumen von ≥ 0,01 ml/g für Porendurchmesser > 50 nm, vorzugsweise ≥ 0,025 ml/g für Porendurchmesser > 100 nm und insbesondere ≥ 0,05 ml/g für Porendurchmesser > 200 nm. Weiterhin liegt das Verhältnis von Makroporen mit einem Durchmesser > 50 nm zum Gesamtporenvolumen für Poren mit einem Durchmesser > 4 nm bei Werten > 10 %, bevorzugt > 20 %, insbesondere > 30 %. Oftmals lassen sich durch Verwendung dieser Katalysatoren hohe Pyrrolidon-Ausbeuten und -Selektivitäten erreichen. Die erwähnten Porositäten wurden durch Quecksilber-Intrusion nach DIN 66133 bestimmt. Es wurden die Daten im Porendurchmesserbereich von 4 nm bis 300 µm ausgewertet.

Die erfindungsgemäß verwendeten Katalysatoren besitzen im allgemeinen eine ausreichende Standzeit. Für den Fall, daß die Aktivität und/oder Selektivität des Katalysators dennoch im Laufe ihrer Betriebszeit sinken sollte, kann dieser durch dem Fachmann bekannte Maßnahmen regeneriert werden. Hierzu zählt vorzugsweise eine reduktive Behandlung des Katalysators im Wasserstoffstrom bei erhöhter Temperatur. Gegebenenfalls kann der reduktiven Behandlung eine oxidative vorausgehen. Hierbei wird die Katalysatorschüttung mit einem molekularen Sauerstoff enthaltenden Gasgemisch, beispielsweise Luft, bei erhöhter Temperatur durchströmt. Weiterhin besteht die Möglichkeit, den Katalysator mit einem geeigneten Lösungsmittel, beispielsweise Ethanol, THF oder GBL, zu waschen und anschließend in einem Gasstrom zu trocknen.

Zum Erzielen der erfindungsgemäßen Pyrrolidon-Selektivitäten ist weiterhin das Einhalten gewisser Reaktionsparameter erforderlich.

Ein wichtiger Parameter ist das Einhalten einer geeigneten Reaktionstemperatur. Dies wird zum einen erreicht durch eine genügend hohe Eingangstemperatur der Edukte. Diese liegt bei Werten von 200 bis 300°C, vorzugsweise 210 bis 280°C.

Die Katalysatorbelastung der erfindungsgemäßen Hydrierung liegt im Bereich von 0,01 bis 1,0 kg Edukt/1 Katalysator • Stunde. Bei einer möglichen, jedoch nicht bevorzugten Rückführung von durch unvollständige Hydrierung entstandenem Zwischenprodukt, im Fall der Verwendung von MSA als Edukt beispielsweise Succinimid oder ein N-substituiertes Succinimid, ist die Katalysatorbelastung die Summe aus frisch zugeführtem Edukt und rückgeführtem Zwischenprodukt. Wird die Katalysatorbelastung über den genannten Bereich hinaus erhöht, ist generell eine Erhöhung des Anteils am Zwischenprodukt im Hydrieraustrag zu beobachten. Vorzugweise liegt die Katalysatorbelastung im Bereich von 0,02 bis 1, insbesondere 0,05 bis 0,5 kg Edukt/l Katalysator • Stunde. Der Begriff "Edukt" umfaßt dabei die in das erfindungsgemäße Verfahren eingesetzten Ausgangsprodukte, bei denen im Verlauf des Verfahrens Funktionen hydriert werden, wie C=O- oder C=C-Doppelbindungen. Eingesetzte Amine oder Ammoniak werden generell von dem Begriff "Edukt" im Zusammenhang mit der Katalysatorbelastung nicht umfaßt. Im Fall der Rückführung wird als Edukt auch zunächst entstehendes Hydrierprodukt verstanden, das dann nach Rückführung weiter zu Produkt hydriert wird, also beispielsweise N-Methylsuccinimid für den Fall des Einsatzes von MSA und Methylamin in die Hydrierreaktion.

Das Wasserstoff/Edukt-Molverhältnis ist ebenfalls ein Parameter, der einen wichtigen Einfluß auf die Produktverteilung und auch die Wirtschaftlichkeit des erfindungsgemäßen Verfahrens hat. Aus wirtschaftlicher Sicht ist ein niedriges Wasserstoff/Edukt-Verhältnis wünschenswert. Die Untergrenze liegt bei einem Wert von 3, wobei jedoch generell höhere Wasserstoff/Edukt-Molverhältnisse von 20 bis 400 angewendet werden. Der Einsatz der oben beschriebenen, erfindungsgemäßen Katalysatoren sowie das Einhalten der oben beschriebenen Temperaturwerte erlaubt den Einsatz günstiger, niedriger Wasserstoff/Edukt-Verhältnisse, die vorzugsweise bei Werten von 20 bis 200, vorzugsweise 40 bis 150 liegen. Der günstigste Bereich liegt bei Werten von 50 bis 100.

Um die erfindungsgemäß verwendeten Wasserstoff/Edukt-Molverhältnisse einzustellen, wird ein Teil, vorteilhafterweise die Hauptmenge, des Wasserstoffs im Kreis gefahren. Hierzu setzt man im allgemeinen die dem Fachmann bekannten Kreisgasverdichter ein.

Die chemisch durch die Hydrierung verbrauchte Wasserstoffmenge wird ergänzt. In einer bevorzugten Ausführungsform wird ein Teil des Kreisgases ausgeschleust, um Inertverbindungen, beispielsweise n-Butan, zu entfernen. Der im Kreis geführte Wasserstoff kann auch, gegebenenfalls nach Vorheizen, zum Verdampfen des Eduktstroms benutzt werden.

Das Molverhältnis der Edukte C₄-Dicarbonsäure und/oder deren Derivat zu Ammoniak bzw. primärem Amin liegt bei 1 zu 5, bevorzugt 1 zu 3, besonders bevorzugt 1 zu 1,5.

Es kann vorteilhaft sein, bei der Gasphasen-Hydrierung ein Lösungsmittel mitzuverwenden. Hierfür kommen z.B. Ether wie Dioxan, Tetrahydrofuran, Alkohole wie Methanol oder Kohlenwasserstoffe wie Cyclohexan in Frage.

Auch der Volumenstrom der Reaktionsgase, generell ausgedrückt als GHSV (Gas Hourly Space Velocity) ist eine wichtige Größe des erfindungsgemäßen Verfahrens. Die GHSV-Werte des erfindungsgemäßen Verfahrens liegen bei Werten von 100 bis 10.000 Nm³/m³h, vorzugsweise 1000 bis 3000 Nm³/m³h, insbesondere 1100 bis 2500 Nm³/m³h.

Der Druck, bei dem die erfindungsgemäße Hydrierung durchgeführt wird, liegt bei Werten von 1 bis 100 bar, vorzugsweise 1 bis 50 bar, insbesondere 1 bis 20 bar.Gemeinsam mit dem Wasserstoff-Kreisgas werden alle Produkte im Kreis geführt, die beim Kühlen des aus dem Hydrierreaktor austretenden Gasstroms nicht oder nicht vollständig auskondensieren. Dies sind vor allem Wasser, Ammoniak und Amine und Nebenprodukte wie Methan und Butan. Die Kühltemperatur beträgt 0 bis 60 °C, vorzugsweise 20 bis 45 °C.

Als Reaktortypen kommen alle, für heterogen katalysierte Reaktionen mit einem gasförmigen Edukt- und Produktstrom geeigneten Apparate in Betracht. Bevorzugt sind Rohrreaktoren, Schachtreaktoren oder Reaktoren mit innerer Wärmeabfuhr, beispielsweise Rohrbündelreaktoren, es ist auch der Einsatz eines Wirbelbetts möglich. Besonders bevorzugt eingesetzt werden Rohrbündelreaktoren. Es können mehrere Reaktoren parallel oder hintereinander geschaltet eingesetzt werden. Prinzipiell kann zwischen die Katalysatorbetten eine Zwischeneinspeisung erfolgen. Möglich ist auch eine Zwischenkühlung zwischen oder in den Katalysatorbetten. Bei Einsatz von Festbettreaktoren ist eine Verdünnung des Katalysators durch Inertmaterial möglich.

Der aus dem Reaktor austretende Gasstrom wird auf 10 bis 60°C gekühlt. Dabei werden die Reaktionsprodukte auskondensiert und in einen Abscheider geleitet. Der nichtkondensierte Gasstrom wird vom Abscheider abgezogen und dem Kreisgasverdichter zugeführt. Eine kleine Kreisgasmenge wird ausgeschleust. Die auskondensierten Reaktionsprodukte werden dem System kontinuierlich entnommen und der Aufarbeitung zugeführt, die beispielsweise durch Destillation erfolgen kann.

Das erfindungsgemäße Verfahren zeichnet sich dadurch aus, daß zu hydrierende Edukte unterschiedlicher Reinheit in die Hydrierreaktion eingesetzt werden können. Selbstverständlich kann ein Edukt hoher Reinheit, insbesondere MSA, in die Hydrierreaktion eingesetzt werden. Der erfindungsgemäß verwendete Katalysator sowie die sonstigen erfindungsgemäß gewählten Reaktionsbedingungen ermöglichen aber auch den Einsatz von Edukten geringer Reinheit, insbesondere MSA, das mit den üblichen, bei der Oxidation von Benzol, Butenen oder n-Butan anfallenden Verbindungen sowie eventuell weiteren Komponenten verunreinigt ist. Somit kann das erfindungsgemäße Verfahren in einer weiteren Ausführungsform eine vorgeschaltete Stufe umfassen, die das Herstellen der C₄-Dicarbonsäure und/oder des Derivats davon durch partielle Oxidation eines geeigneten Kohlenwasserstoffs sowie das Abtrennen dieses Edukts aus dem damit erhaltenen Produktstrom umfaßt.

Insbesondere ist die C₄-Dicarbonsäure MSA. Dabei wird bevorzugt MSA eingesetzt, welches aus der Partialoxidation von Kohlenwasserstoffen stammt. Geeignete Kohlenwasserstoffströme sind Benzol, C₄-Olefine (z.B. n-Butene, C₄-Raffinatströme) oder n-Butan. Besonders bevorzugt eingesetzt wird n-Butan, da es einen preiswerten, wirtschaftlichen Einsatzstoff darstellt. Verfahren zur Partialoxidation von n-Butan sind beispielsweise in Ullmann's Encyclopedia of Industrial Chemistry, 6^{th} Edition, Electronic Release, Maleic and Fumaric Acids - Maleic Anhydride beschrieben.

Der so erhaltene Reaktionsaustrag wird dann in einem geeigneten organischen Lösungsmittel oder -gemisch aufgenommen, das bei Atmosphärendruck einen um mindestens 30°C höheren Siedepunkt als MSA hat.

Dieses Lösungsmittel (Absorptionsmittel) wird auf eine Temperatur im Bereich zwischen 20 und 160 °C, bevorzugt zwischen 30 und 80 °C, gebracht. Der Maleinsäureanhydrid enthaltende Gasstrom aus der Partialoxidation kann in vielfältiger Weise mit dem Lösungsmittel in Kontakt gebracht werden: (i) Einleiten des Gasstroms in das Lösungsmittel (z.B. über Gaseinleitungsdüsen oder Begasungsringe), (ii) Einsprühen des Lösungsmittels in den Gasstrom und (iii) Gegenstromkontakt zwischen dem nach oben strömenden Gasstrom und dem nach unten strömenden Lösungsmittel in einer Boden- oder Packungskolonne. In allen drei Varianten können die dem Fachmann bekannten Apparate zur Gasabsorption eingesetzt werden. Bei der Wahl des einzusetzenden Lösungsmittels ist darauf zu achten, daß dies nicht mit dem Edukt, beispielsweise dem vorzugsweise eingesetzten MSA, reagiert. Geeignete Lösungsmittel sind: Trikresylphosphat, Dibutylmaleat, hochmolekulare Wachse, aromatische Kohlenwasserstoffe mit einem Molekulargewicht zwischen 150 und 400 und einem Siedepunkt oberhalb 140 °C, wie beispielsweise Dibenzylbenzol; Dialkylphthalate mit C₁-C₈-Alkylgruppen, beispielsweise Dimethylphthalat, Diethylphthalat, Dibutylphthalat, Di-n-Propyl-und Di-iso-Propylphthalat; Di-C₁-C₄-Alkylester anderer aromatischer und aliphatischer Dicarbonsäuren, beispielsweise Dimethyl-2,3-Naphthalin-Dicarbonsäure, Dimethyl-1,4-Cyclohexan-Dicarbonsäure, Methylester langkettiger Fettsäuren mit beispielsweise 14 bis 30 Kohlenstoffatomen, hochsiedende Ether, beispielsweise Dimethylether von Polyethylenglykol, beispielsweise Tetraethylenglykoldimethylether.

Der Einsatz von Phthalaten ist bevorzugt.

Die nach der Behandlung mit dem Absorptionsmittel resultierende Lösung hat generell einen MSA-Gehalt von etwa 5 bis 400 Gramm pro Liter.

Es ist nach der vorliegenden Erfindung auch in einer bevorzugten Ausführungsform, gemäß WO 01/27.058 A1 möglich, mit dem Reaktionsaustrag eine Partialkondensation durchzuführen, das flüssig abgeschiedene MSA ohne weitere Reinigung für die erfindungsgemässe Hydrierung zu verwenden und das restliche, in der Gasphase gebliebene MSA, wie vorstehend beschrieben, in einem organischen Lösungsmittel aufzunehmen und gegebenenfalls dieses MSA, nach Entfernen des Lösungsmittels, in die Hydrierung einzusetzen. Das Verfahren der partiellen Kondensation/Extraktion von MSA und Einspeisen des MSA in die nachfolgende Reaktionsstufe wie in WO 01/27.058 beschrieben ist ein integraler Bestandteil der vorliegenden Anmeldung und durch Bezugnahme in diese eingeschlossen.

Der nach der Behandlung mit dem Absorptionsmittel verbleibende Abgasstrom enthält hauptsächlich die Nebenprodukte der vorangegangenen Partialoxidation, wie Wasser, Kohlenmonoxid, Kohlendioxid, nicht umgesetzte Butane, Essig- und Acrylsäure. Der Abgasstrom ist praktisch frei von MSA.

Anschließend wird das gelöste MSA aus dem Absorptionsmittel ausgetrieben. Dies erfolgt mit Wasserstoff bei oder maximal 10% oberhalb des Druckes der anschließenden Hydrierung oder alternativ im Vakuum mit anschließender Kondensation von verbleibendem MSA. In der Strippkolonne wird ein Temperaturprofil beobachtet, das sich aus den Siedepunkten von MSA am Kopf und dem nahezu MSA-freien Absorptionsmittel am Sumpf der Kolonne bei dem jeweiligen Kolonnendruck und der eingestellten Verdünnung mit Trägergas (im ersten Fall mit Wasserstoff) ergibt. Im Fall der Direktstrippung mit Wasserstoff wird bei einer Kopftemperatur von 130°C und einem Druck von 5 bar gearbeitet.

Um Verluste an Lösungsmittel zu verhindern, können sich oberhalb der Zufuhr des Roh-MSA-Stromes Rektifiziereinbauten befinden. Das vom Sumpf abgezogene, nahezu MSA-freie Absorptionsmittel wird wieder der Absorptionszone zugeführt. Im Fall der Direktstrippung mit Wasserstoff wird vom Kopf der Kolonne ein nahezu gesättigter Gasstrom von MSA in Wasserstoff mit einer Temperatur von 180 °C und einem Druck von 5 bar abgezogen. Das H₂/MSA-Verhältnis liegt bei etwa 20 bis 400. Im anderen Fall wird das kondensierte MSA in einen Verdampfer gepumpt und dort in den Kreisgasstrom verdampft.

Der MSA-Wasserstoff-Strom enthält noch Nebenprodukte, die bei der partiellen Oxidation von n-Butan, Butenen oder Benzol mit Sauerstoff enthaltenden Gasen entstehen, sowie nicht abgetrenntes Absorptionsmittel. Hierbei handelt es sich vor allem um Essigsäure und Acrylsäure als Nebenprodukte, Wasser, Maleinsäure sowie die vorzugsweise als Absorptionsmittel verwendeten Dialkylphthalate. Das MSA enthält Essigsäure in Mengen von 0,01 bis 1 Gew.-%, vorzugsweise 0, 1 bis 0,8 Gew.-% und Acrylsäure in Mengen von 0,01 bis 1 Gew.-%, vorzugsweise 0,1 bis 0,8 Gew.-%, bezogen auf MSA. In der Hydrierstufe werden Essigsäure und Acrylsäure ganz oder teilweise zu Ethanol bzw. Propanol hydriert. Der Maleinsäure-Gehalt beträgt 0,01 bis 1 Gew.-%, insbesondere 0,05 bis 0,3 Gew.-%, bezogen auf MSA.

Werden Dialkylphthalate als Absorptionsmittel eingesetzt, hängt deren Gehalt im MSA stark vom richtigen Betrieb der Strippkolonne, insbesondere vom Verstärkungsteil ab. Phthalatgehalte von bis 1,0 Gew.-% , insbesondere bis 0,5 Gew.-% sollten bei geeigneter Betriebsweise nicht überschritten werden, da sonst der Verbrauch an Absorptionsmittel zu hoch wird.

Der so erhaltene Wasserstoff/Maleinsäureanhydrid-Strom wird nun mit Ammoniak bzw. einem primären Amin versetzt und mit dem erfindungsgemäßen Verfahren unter Hydrierung zu dem gewünschten, gegebenenfalls N-substituierten Pyrrolidon umgesetzt.

Das erfindungsgemäße Verfahren wird nun in den nachfolgenden Beispielen näher erläutert.

### Beispiel 1 Herstellung von N-Methylpyrrolidon

### a) Katalysator-Aktivierung

Der in den Beispielen 1 bis0 3 verwendete Katalysator besteht vor der Aktivierung aus 50 Gew.-% CuO und 50 Gew.-% Al₂O₃. Er wird vor Reaktionsbeginn einer Wasserstoffbehandlung bei 180°C unterzogen. Der Katalysator wird dabei nacheinander die in Tabelle 1 angegebene Zeit mit dem jeweils angegebenen Gemisch aus Wasserstoff und Stickstoff bei Atmosphärendruck aktiviert.

**Tabelle 1**

| **Zusammensetzung** | **Zeit (Minuten)** | **Wasserstoff (Nl/h)** | **Stickstoff (Nl/h)** |
|---|---|---|---|
| 50 Gew.-%/CuO, 50 Gew.-%/Al₂O₃ | 120 | 10 | 550 |
| | 30 | 25 | 400 |
| | 15 | 60 | 100 |
| | 180 | 60 | 0 |

### b) Hydrierapparatur und Durchführung der Hydrierung:

Die kontinuierlich betriebene Hydrierung wird in einem senkrecht stehenden, elektrisch beheizten Rohrreaktor aus Quarz durchgeführt, der den aktivierten Katalysator in Form von 3 x 3 mm Tabletten enthält. Maleinsäureanhydrid wird von oben als Schmelze zugeführt, Methylamin wird zusammen mit Wasserstoff gasförmig ebenfalls von oben auf den Katalysator geleitet. Die am unteren Ende des Reaktors austretenden Produkte werden gekühlt. Der flüssige Austrag wird gaschromatographisch analysiert. Die Ausgangsprodukte, Reaktionsbedingungen, Ausbeuten, Umsätze und Selektivitäten sind in Tabelle 2 zusammengefasst. Das Molverhältnis von Maleinsäureanhydrid zu Wasserstoff beträgt 1:10.

**Tabelle 2**

| **Beispiel** | **Ausgangsverbindung** | **Katalysator [Gew.- %]**^{**2)**} | **Katalysator- Belastung [kg/l Kat-h]** | **Molverhältnis MSA/ CH**_{**3**}**NH**_{**2**} | **Temperatur [°C]** | **Druck [bar]** | **NMP-Ausbeute** ^{**1)**} **[%]** | **Umsatz [%]** | **NMP-Selektivität** |
|---|---|---|---|---|---|---|---|---|---|
| 1 | Maleinsäureanhydrid | 50 Gew.-% CuO, 50 Gew.-% Al₂O₃ | 0,1 | 1:1,5 | 250 | 1 | 70 | 100 | 70 |
| 2 | Bernsteinsäureanhydrid | s.o. | 0,1 | 1:1,5 | 245 | 1 | 65 | 70 | 93 |
| 3 | N-Methylsuccinimid | s.o. | 0,1 | kein CH₃NH₂ | 245 | 1 | 90 | 95 | 95 |

| | | | | | | | | | |
|---|---|---|---|---|---|---|---|---|---|
| ¹⁾ N-Methylpyrrolidon | | | | | | | | | |
| ²⁾ vor Aktivierung mit Wasserstoff | | | | | | | | | |

## Patentansprüche

1. Verfahren zur Herstellung von gegebenenfalls N-substituiertem Pyrrolidon durch Hydrierung in der Gasphase unter wasserfreien Bedingungen eines Substrats, das ausgewählt ist aus C₄-Dicarbonsäuren und deren Derivaten, gegebenenfalls unter Zusatz von Ammoniak oder primären Aminen, unter Verwendung eines Cr-freien Katalysators, der 5 bis 95 Gew.-% CuO, vorzugsweise 30 bis 70 Gew.-% CuO, und 5 bis 95 Gew.-% Al₂O₃, vorzugsweise 30 bis 70 Gew.-% Al₂O₃, und 0 bis 60 Gew.-%, vorzugsweise 5 bis 40 Gew.-% ZnO aufweist.

2. Verfahren nach Anspruch 1, **dadurch gekennzeichnet, daß** der Katalysator ein oder mehrere weitere Metalle oder eine Verbindung davon, vorzugsweise ein Oxid, aus den Gruppen 1 bis 14 des Periodensystems der Elemente enthält, vorzugsweise eine Verbindung aus der Gruppe bestehend aus TiO₂, ZrO₂, SiO₂ und MgO.

3. Verfahren nach Anspruch 1 oder 2, **dadurch gekennzeichnet, daß** die C₄-Dicarbonsäure bzw. das Derivat davon ausgewählt ist aus der Gruppe bestehend aus Maleinsäure, Maleinsäureanhydrid, Bernsteinsäure, Bernsteinsäureanhydrid und gegebenenfalls substituiertem Maleinimid und Succinimid, insbesondere Maleinsäureanhydrid.

4. Verfahren nach einem der Ansprüche 1 bis 3, **dadurch gekennzeichnet, daß** das primäre Amin oder das Imid einen Substituenten aufweist, der ausgewählt ist aus der Gruppe bestehend aus gegebenenfalls substituierten cyclischen und acyclischen, verzweigten und unverzweigten aliphatischen C₁-C₂₀-Kohlenwasserstoffen, vorzugsweise C₁-C₁₂-Kohlenwasserstoffgruppen.

5. Verfahren nach einem der Ansprüche 1 bis 4, **dadurch gekennzeichnet, daß** die aliphatischen Gruppen einen oder mehrere Substituenten aus der Gruppe bestehend aus Phenylgruppen, Halogenen, Hydroxylgruppen und Alkoxygruppen aufweisen können, vorzugsweise die aliphatische Gruppe ausgewählt ist aus Methyl, Ethyl, n-Propyl, n-Butyl, n-Decyl, n-Dodecyl, Cyclohexyl, Benzyl und Ethylol.

6. Verfahren nach einem der Ansprüche 1 bis 5, **dadurch gekennzeichnet, daß** eine ungesättigte Dicarbonsäure oder ein Derivat davon, vorzugsweise Maleinsäure, ein Maleinsäureester und/oder Maleinsäureanhydrid als Edukt eingesetzt werden und der Ammoniak oder das primäre Amin dem Reaktionsgemisch erst nach teilweisem oder vollständigem Hydrieren der olefinischen Doppelbindung des oder der Edukte zugegeben wird.

7. Verfahren nach einem der Ansprüche 1 bis 6, **dadurch gekennzeichnet, daß** die Reaktion bei Temperaturen von 200 bis 300°C, vorzugsweise 210 bis 280°C, durchgeführt wird.

8. Verfahren nach einem der Ansprüche 1 bis 7, **dadurch gekennzeichnet, daß** das Verfahren bei Drücken von 1 bis 100 bar, vorzugsweise 1 bis 50 bar, durchgeführt wird.

9. Verfahren nach einem der Ansprüche 1 bis 8, **dadurch gekennzeichnet, daß** ein Festbettreaktor, vorzugsweise ein Rohreaktor, ein Schachtreaktor, ein Wirbelbettreaktor oder ein Reaktor mit innerer Wärmeabfuhr, insbesondere ein Rohrbündelreaktor, eingesetzt wird.

10. Verfahren nach einem der Ansprüche 1 bis 9, **dadurch gekennzeichnet, daß** Maleinsäureanhydrid eingesetzt wird, das durch Oxidation von Benzol, C₄-Olefinen oder n-Butan hergestellt wurde, wobei das durch Oxidation erhaltene Roh-Maleinsäureanhydrid mit einem Lösungsmittel aus dem Rohproduktgemisch extrahiert und anschließend aus diesem Lösungsmittel mit Wasserstoff ausgetrieben wurde.

11. Verfahren nach Anspruch 10, **dadurch gekennzeichnet, dass** aus dem durch Oxidation erhaltenen Rohproduktgemisch Maleinsäureanhydrid auskondensiert und ohne Reinigung in die Hydrierung eingesetzt wird und danach das nicht kondensierte Maleinsäureanhydrid mit einem Lösungsmittel aus dem Rohproduktgemisch extrahiert und gegebenenfalls für die Hydrierung verwendet wird.

12. Verfahren nach Anspruch 1, **dadurch gekennzeichnet, dass** ein Gemisch aus primärem Amin mit dem entsprechenden sekundären und tertiären Amin eingesetzt wird.

## Claims

1. A process for preparing pyrrolidone, which may be N-substituted, by hydrogenation in the gas phase under anhydrous conditions of a substrate selected from among C₄-dicarboxylic acids and their derivatives, with or without addition of ammonia or primary amines and using a Cr-free catalyst which comprises from 5 to 95% by weight of CuO, preferably from 30 to 70% by weight of CuO, and from 5 to 95% by weight of Al₂O₃, preferably from 30 to 70% by weight of Al₂O₃, and from 0 to 60% by weight, preferably from 5 to 40% by weight, of ZnO.

2. A process as claimed in claim 1, wherein the catalyst comprises one or more metals or compounds thereof, preferably an oxide, from groups 1 to 14 of the Periodic Table of the Elements, preferably a compound selected from the group consisting of TiO₂, ZrO₂, SiO₂ and MgO.

3. A process as claimed in claim 1 or 2, wherein the C₄-dicarboxylic acid or the derivative thereof is selected from the group consisting of maleic acid, maleic anhydride, succinic acid, succinic anhydride and substituted and unsubstituted maleimide and succinimide, in particular maleic anhydride.

4. A process as claimed in any of claims 1 to 3, wherein the primary amine or the imide bears a substituent selected from the group consisting of substituted and unsubstituted cyclic and acyclic, branched and unbranched aliphatic C₁-C₂₀-hydrocarbons, preferably C₁-C₁₂-hydrocarbon groups.

5. A process as claimed in any of claims 1 to 4, wherein the aliphatic groups may bear one or more substituents selected from the group consisting of phenyl groups, halogens, hydroxyl groups and alkoxy groups, and the aliphatic groups are preferably selected from among methyl, ethyl, n-propyl, n-butyl, n-decyl, n-dodecyl, cyclohexyl, benzyl and ethylol.

6. A process as claimed in any of claims 1 to 5, wherein an unsaturated dicarboxylic acid or a derivative thereof, preferably maleic acid, a maleic ester and/or maleic anhydride, is used as starting material and the ammonia or the primary amine is added to the reaction mixture only after partial or complete hydrogenation of the olefinic double bond of the starting material or materials.

7. A process as claimed in any of claims 1 to 6, wherein the reaction is carried out at from 200 to 300°C, preferably from 210 to 280°C.

8. A process as claimed in any of claims 1 to 7 carried out at pressures of from 1 to 100 bar, preferably from 1 to 50 bar.

9. A process as claimed in any of claims 1 to 8 in which a fixed-bed reactor, preferably a tube reactor, a shaft reactor, a fluidized-bed reactor or a reactor with internal removal of heat, in particular a shell-and-tube reactor, is used.

10. A process as claimed in any of claims 1 to 9, wherein maleic anhydride is obtained by oxidation of benzene, C₄-olefins or n-butane, extraction of the maleic anhydride by means of a solvent from the crude product mixture obtained by oxidation and subsequent stripping from this solvent by means of hydrogen is used.

11. A process as claimed in claim 10, wherein maleic anhydride is condensed from the crude product mixture obtained by oxidation and is used without purification in the hydrogenation and the uncondensed maleic anhydride is then extracted from the crude product mixture by means of a solvent and is optionally used for the hydrogenation.

12. A process as claimed in claim 1, wherein a mixture of primary amine with the corresponding secondary and tertiary amine is used.

## Revendications

1. Procédé pour la préparation de pyrrolidone, le cas échéant substituée, par hydrogénation en phase gazeuse, sous des conditions anhydres, d'un substrat choisi parmi les acides dicarboxyliques en C₄ et leurs dérivés, le cas échéant avec addition d'ammoniaque ou d'amines primaires, avec utilisation d'un catalyseur exempt de Cr, qui présente 5 à 95% en poids de CuO, de préférence 30 à 70% en poids de CuO, et 5 à 95% en poids d'Al₂O₃, de préférence 30 à 70% en poids d'Al₂O₃, et 0 à 60% en poids, de préférence 5 à 40% en poids de ZnO.

2. Procédé selon la revendication 1, **caractérisé en ce que** le catalyseur contient un ou plusieurs autres métaux ou un composé de ceux-ci, de préférence un oxyde, des groupes 1 à 14 du système périodique des éléments, de préférence un composé du groupe constitué par TiO₂, ZrO₂, SiO₂ et MgO.

3. Procédé selon la revendication 1 ou 2, **caractérisé en ce que** l'acide dicarboxylique en C₄ ou le dérivé de celui-ci est choisi dans le groupe constitué par l'acide maléique, l'anhydride de l'acide maléique, l'acide succinique, l'anhydride de l'acide succinique et le cas échéant le maléinimide et le succinimide, le cas échéant substitués, en particulier l'anhydride de l'acide maléique.

4. Procédé selon l'une quelconque des revendications 1 à 3, **caractérisé en ce que** l'amine primaire ou l'imide présente un substituant qui est choisi dans le groupe constitué par les hydrocarbures en C₁ à C₂₀, de préférence les groupes hydrocarbonés en C₁ à C₁₂, aliphatiques, cycliques et acycliques, ramifiés et non ramifiés, le cas échéant substitués.

5. Procédé selon l'une quelconque des revendications 1 à 4, **caractérisé en ce que** les groupes aliphatiques peuvent présenter un ou plusieurs substituants du groupe constitué par les groupes phényle, les halogènes, les groupes hydroxyle et les groupes alcoxy, le groupe aliphatique étant de préférence choisi parmi méthyle, éthyle, n-propyle, n-butyle, n-décyle, n-dodécyle, cyclohexyle, benzyle et éthylol.

6. Procédé selon l'une quelconque des revendications 1 à 5, **caractérisé en ce qu'**un acide dicarboxylique insaturé ou un dérivé de celui-ci, de préférence l'acide maléique, un ester de l'acide maléique et/ou l'anhydride de l'acide maléique sont utilisés comme produit de départ et l'ammoniaque ou l'amine primaire n'est ajoutée au mélange réactionnel qu'après une hydrogénation partielle ou complète de la double liaison oléfinique du ou des produits de départ.

7. Procédé selon l'une quelconque des revendications 1 à 6, **caractérisé en ce que** la réaction est réalisée à des températures de 200 à 300°C, de préférence de 210 à 280°C.

8. Procédé selon l'une quelconque des revendications 1 à 7, **caractérisé en ce que** le procédé est réalisé à des pressions de 1 à 100 bars, de préférence de 1 à 50 bars.

9. Procédé selon l'une quelconque des revendications 1 à 8, **caractérisé en ce qu'**on utilise un réacteur à lit fixe, de préférence un réacteur tubulaire, un réacteur à cuve, un réacteur à lit fluidisé ou un réacteur avec une évacuation de chaleur interne, en particulier un réacteur à faisceau tubulaire.

10. Procédé selon l'une quelconque des revendications 1 à 9, **caractérisé en ce qu'**on utilise de l'anhydride de l'acide maléique qui a été préparé par oxydation de benzène, d'oléfines en C₄ ou de n-butane, l'anhydride de l'acide maléique brut obtenu par oxydation étant extrait du mélange de produits bruts avec un solvant et ensuite chassé de ce solvant avec de l'hydrogène.

11. Procédé selon la revendication 10, **caractérisé en ce que** l'anhydride de l'acide maléique est éliminé par condensation du mélange de produits bruts obtenu par oxydation et utilisé sans purification dans l'hydrogénation et l'anhydride de l'acide maléique qui n'a pas été éliminé par condensation est ensuite extrait du mélange de produits bruts avec un solvant et le cas échéant utilisé pour l'hydrogénation.

12. Procédé selon la revendication 1, **caractérisé en ce qu'**on utilise un mélange d'une amine primaire avec l'amine secondaire et tertiaire correspondante.
